# EUROPEAN PATENT SPECIFICATION

(11) **EP 2 985 351 B1**
(45) Date of publication and mention of the grant of the patent: **04.10.2017**
(21) Application number: 14181091.1
(22) Date of filing: 14.08.2014
(51) Int. Cl.: C12Q 1/68

(54) **A CIRCULATING NON-CODING RNA AS PREDICTOR OF MORTALITY IN PATIENTS WITH ACUTE KIDNEY INJURY**
EINE ZIRKULIERENDE NICHT-KODIERENDE RNA ZUR VORHERSAGE DER STERBLICHKEIT DER PATIENTEN MIT AKUTEM NIERENVERSAGEN
UN ARN NON-CODANT CIRCULANT POUR PROGNOSTIQUER LA MORTALITÉ D'UN PATIENT AVEC DÉFAILLANCE RÉNALE

(43) Date of publication of application: 17.02.2016
(73) Proprietor: Medizinische Hochschule Hannover, 30625 Hannover (DE)
(72) Inventor: THUM, Thomas, 30627 Hannover (DE); LORENZEN, Johan, 30539 Hannover (DE)
(74) Representative: Vossius & Partner Patentanwälte Rechtsanwälte mbB

(56) References cited:
- EP-A2- 2 505 667
- US-A1- 2013 178 428
- Anonymous: "Arraystar Human Lnc RNA microarray V2 .0 (Agilent_033010 Probe Name version)", , 6 March 2012 (2012-03-06), XP055130607, Retrieved from the Internet: URL:http://www.ncbi.nlm.nih.gov/geo/query/ acc.cgi?acc=GPL15314 [retrieved on 2014-07-21]
- Arraystar ET AL: "LncRNA and Coding Transcript Collection - Arraystar Human LncRNA Microarray V2", , 1 March 2011 (2011-03-01), XP055130605, Retrieved from the Internet: URL:http://www.arraystar.com/manage/Upload File/201228192230918.pdf [retrieved on 2014-07-21]
- DATABASE Geneseq [Online] 2 April 2009 (2009-04-02), "Human ADHD associated gene SEQ ID NO:12118.", XP002734499, retrieved from EBI accession no. GSN:AWC72366 Database accession no. AWC72366 & WO 2008/118258 A2 (GENIZON BIOSCIENCES INC [CA]; BELOUCHI ABDELMAJID [CA]; RAELSON JOHN V) 2 October 2008 (2008-10-02)
- DATABASE EMBL [Online] 23 October 2013 (2013-10-23), "TPA: Homo sapiens long non-coding RNA OTTHUMT00000321306.1 (RP5-1104E15.6 gene), antisense", XP002734500, retrieved from EBI accession no. EM_STD:HG512306 Database accession no. HG512306
- J. M. LORENZEN ET AL: "Circulating miR-210 Predicts Survival in Critically Ill Patients with Acute Kidney Injury", CLINICAL JOURNAL OF THE AMERICAN SOCIETY OF NEPHROLOGY, vol. 6, no. 7, 23 June 2011 (2011-06-23), pages 1540-1546, XP55044638, ISSN: 1555-9041, DOI: 10.2215/CJN.00430111
- J. SAIKUMAR ET AL: "Expression, Circulation, and Excretion Profile of MicroRNA-21, -155, and -18a Following Acute Kidney Injury", TOXICOLOGICAL SCIENCES, vol. 129, no. 2, 1 October 2012 (2012-10-01), pages 256-267, XP055162004, ISSN: 1096-6080, DOI: 10.1093/toxsci/kfs210
- YA-FENG LI ET AL: "MicroRNA-21 in the pathogenesis of acute kidney injury", PROTEIN & CELL, vol. 4, no. 11, 1 November 2013 (2013-11-01), pages 813-819, XP055161934, ISSN: 1674-800X, DOI: 10.1007/s13238-013-3085-y
- M. LUCRECIA ALVAREZ ET AL: "Role of MicroRNA 1207-5P and Its Host Gene, the Long Non-Coding RNA Pvt1, as Mediators of Extracellular Matrix Accumulation in the Kidney: Implications for Diabetic Nephropathy", PLOS ONE, vol. 8, no. 10, 25 October 2013 (2013-10-25), page e77468, XP055162114, DOI: 10.1371/journal.pone.0077468
- Y. HUANG ET AL: "Non-coding RNAs and diseases", MOLECULAR BIOLOGY, vol. 47, no. 4, 1 July 2013 (2013-07-01), pages 465-475, XP055161923, ISSN: 0026-8933, DOI: 10.1134/S0026893313040171

### SEQUENCE LISTING

<110> Medizinische Hochschule Hannover
<120> A circulating non-coding RNA as predictor of mortality in patients
   with acute kidney injury
<130> X2974 EP
<160> 7
<170> BiSSAP 1.3
<210> 1
   <211> 498
   <212> DNA
   <213> Homo sapiens
<220>
   <223> ENST00000432239
<400> 1
   gctactggtg gtcatcgggg acgcagatat gtgtgtctcc attttgcaga tgaggacatg      60
   gaggcctcaa gaggttaagt gacttgctca gccacacaca gctgatggga atgaaacaga     120
   cggaaacgga gactccccaa gctgacaggc ggactacaga tctgggggtt ctgctccagg     180
   ctcaacacaa gtggcctggg cgagggaggg cctggtagac catgacaccc agcatcgagg     240
   aattctgctt ccccagcagg ctcagaagga agaagtctgc ttcaggggcc aggcctggtc     300
   aaagactcca tcttccctgg atggccagag tcaaatcctt gcaaatcccg ttcaccatgg     360
   agcagagacg agacctatgt tacccaggct gtatgtgaat tcctggactc aagcaatcct     420
   cccatctcag cctcgtgagt agttgggaca agtctcacac caccataccc agcttaattt     480
   aatttgaatt ttaatagc                                                   498
<210> 2
   <211> 20
   <212> DNA
   <213> Artificial Sequence
<220>
   <223> Forward primer ASHGA5P037701
<400> 2
   gtagaccatg acacccagca 20
<210> 3
   <211> 21
   <212> DNA
   <213> Artificial Sequence
<220>
   <223> Reverse primer ASHGA5P037701
<400> 3
   ctcgtctctg ctccatggtg a 21
<210> 4
   <211> 20
   <212> DNA
   <213> Artificial Sequence
<220>
   <223> Forward primer ASHGA5P044509
<400> 4
   aggaaggtga ccatgagaga 20
<210> 5
   <211> 20
   <212> DNA
   <213> Artificial Sequence
<220>
   <223> Reverse primer ASHGA5P044509
<400> 5
   tgcactgaac aagaaaggct 20
<210> 6
   <211> 20
   <212> DNA
   <213> Artificial Sequence
<220>
   <223> Forward primer ASHGA5P034969
<400> 6
   gggtccaaca gtgaagaacg 20
<210> 7
   <211> 20
   <212> DNA
   <213> Artificial Sequence
<220>
   <223> Reverse primer ASHGA5P034969
<400> 7
   tgttaacttt gtggcaggca 20

## Description

The present invention relates to a method for predicting mortality of a test patient with acute kidney injury comprising (a) detecting the expression level of the non-coding RNA (ncRNA) of SEQ ID NO: 1 in a blood sample obtained from said test patient with acute kidney injury, (b) comparing said expression level of the ncRNA with the expression level of this ncRNA in a blood sample obtained from control patients with acute kidney injury or with a predetermined standard expression level of this ncRNA which was obtained on the basis of a blood sample from control patients with acute kidney injury, wherein the control patients were alive at least about 4 weeks after diagnosis of the acute kidney injury, and a greater than 1.5-fold overexpression of the ncRNA in the test patient's blood sample as compared to the control patient's blood sample or as compared to the predetermined standard is indicative of an enhanced likelihood for future death of the test patient due to renal failure; and/or (b') comparing said expression level of the ncRNA with the expression level of this ncRNA in a blood sample obtained from control patients with acute kidney injury or with a predetermined standard expression level of this ncRNA which was obtained on the basis of a blood sample from control patients with acute kidney injury, wherein the control patients died from renal failure within about 4 weeks after diagnosis of acute kidney injury, and a greater than 1.5-fold underexpression of at the ncRNA in the test patient's blood sample as compared to the control patient's blood sample or as compared to the predetermined standard is indicative of an enhanced likelihood for the long term survival of the test patient; and/or (b") comparing said expression level of the ncRNA with the expression level of the ncRNA in a blood sample obtained from a healthy subject or with a predetermined standard expression level of this ncRNA which was obtained on the basis of a at least one blood from a healthy subject, wherein a greater than 5-fold overexpression of the ncRNA in the test patient's blood as compared to the healthy blood sample or as compared to the predetermined standard is indicative of an enhanced likelihood for future death of the test patient due to renal failure.

In this specification, a number of documents including patent applications and manufacturer's manuals is cited. EP 2 505 667 discloses a method for analysis of kidney status in a sample of body fluid by determining the concentration of miR-210. Acute kidney injury (AKI) in critically ill patients has been identified as an independent risk factor for increased mortality [1]. Mortality of patients with AKI in the intensive care unit is still unacceptably high despite significant advances in supportive care [2]. A recent multinational, multi-centre study of 29,000 critically ill patients including 1,700 with AKI revealed that the in-hospital mortality exceeds 60% [3].

Thus, there is an ongoing need for new means and methods for the detection of patients at particular risk for both, death and prolonged kidney failure after AKI, e.g. in the setting of intensive care medicine and/or renal replacement therapy (RRT). This need is addressed by the present invention. The sample used in the methods of the present invention as described in the following is a blood sample. Hence, the present invention relates in a first aspect to a method for predicting mortality of a test patient with acute kidney injury comprising (a) detecting the expression level of the non-coding RNA (ncRNA) of SEQ ID NO: 1 in a sample obtained from said test patient with acute kidney injury, (b) comparing said expression level of the ncRNA with the expression level of this ncRNA in a sample obtained from control patients with acute kidney injury or with a predetermined standard expression level of this ncRNA which was obtained on the basis of a sample from control patients with acute kidney injury, wherein the control patients were alive at least about 4 weeks after diagnosis of the acute kidney injury, and a greater than 1.5-fold overexpression of the ncRNA in the test patient's sample as compared to the control patient's sample or as compared to the predetermined standard is indicative of an enhanced likelihood for future death of the test patient due to renal failure; and/or (b') comparing said expression level of the ncRNA with the expression level of this ncRNA in a sample obtained from control patients with acute kidney injury or with a predetermined standard expression level of this ncRNA which was obtained on the basis of a sample from control patients with acute kidney injury, wherein the control patients died from renal failure within about 4 weeks after diagnosis of acute kidney injury, and a greater than 1.5-fold underexpression of at the ncRNA in the test patient's sample as compared to the control patient's sample or as compared to the predetermined standard is indicative of an enhanced likelihood for the long term survival of the test patient; and/or (b") comparing said expression level of the ncRNA with the expression level of the ncRNA in a sample obtained from a healthy subject or with a predetermined standard expression level of this ncRNA which was obtained on the basis of at least one sample from a healthy subject, wherein a greater than 5-fold overexpression of the ncRNA in the test patient's sample as compared to the healthy sample or as compared to the predetermined standard is indicative of an enhanced likelihood for future death of the test patient due to renal failure.

The method uses isolated blood. Accordingly, the method is in general performed in vitro and preferably does not comprise an invasive step for obtaining the sample from a patient and healthy subject.

A patient having an enhanced likelihood for future death is preferably further treated by intensive care medicine, involving intense medication of AKI and/or renal replacement therapy. On the other hand, a patient having an enhanced likelihood for the long term survival is preferably discharged from intensive care medicine.

The method according to the present disclosure may also encompass detecting and comparing the expression level of one or more ncRNAs being with increased preference at least 90%, at least 92%, at least 94%, at least 96%, at least 98%, at least 99%, and at least 99.5% identical to SEQ ID NO 1, independently of or in conjunction with the detection of the expression level of the ncRNA of SEQ ID NO: 1. Means and methods for determining sequence identity are known in the art. Preferably, the BLAST (Basic Local Alignment Search Tool) program is used for determining the sequence identity with regard to the ncRNA of SEQ ID NO 1. The method disclosed herein may furthermore encompass detecting and comparing the expression level of a ncRNA differing with increasing preference by no more than 10, such as 5, 4, 3, 2 or 1 nucleotide(s) from SEQ ID NO: 1. The nucleotide differences may be the addition, deletion and/or substitution of nucleotide(s). The sequences the expression of which is compared, while being homologous, may also differ from each other with increasing preference by no more than 10, such as 5, 4, 3, 2 or 1 nucleotide(s).

The term "predicting mortality" as used herein defines determining the future likelihood whether a patient suffering from acute kidney injury will die from renal failure or will survive the acute kidney injury (AKI) for a long term. The long term is with increasing preference more than about 4 weeks, more than about 12 weeks, more than about 36 weeks, more than about 1 year and more than about 5 years. In this connection the term "about" is preferably ± 1 week.

The cause of the possible future death of the test patient and the death of the control patients is in accordance with the invention renal failure. Renal failure (also kidney failure or renal insufficiency) is a medical condition in which the kidneys fail to adequately filter waste products from the blood. Healthy kidneys clean the blood of a subject by removing excess fluid, minerals, and wastes. If the kidneys are damaged, they don't function properly. Harmful wastes can build up in the body. The blood pressure may rise. In addition, the body may retain excess fluid and may not produce enough red blood cells.

Acute kidney injury (AKI; previously known as acute renal failure) is a rapidly progressive loss of renal function, generally characterized by oliguria (decreased urine production, quantified as less than 400 mL per day in adults, less than 0.5 mL/kg/h in children or less than 1 mL/kg/h in infants); and fluid and electrolyte imbalance. AKI is an abrupt loss of kidney function that generally develops within 7 days (Mehta RL et al. (2007); Critical Care (London, England) 11 (2): R31.). AKI can result from a variety of causes, generally classified as *prerenal*, *intrinsic*, and *postrenal.* The underlying cause must be identified and treated to arrest the progress, and dialysis may be necessary to bridge the time gap required for treating these fundamental causes. AKI usually occurs when the blood supply to the kidneys is suddenly interrupted or when the kidneys become overloaded with toxins. Causes of acute kidney injury include accidents, injuries, or complications from surgeries in which the kidneys are deprived of normal blood flow for extended periods of time. Heart-bypass surgery is an example of one such procedure. Drug overdoses, accidental or from other chemical overloads of drugs such as antibiotics or chemotherapy, may also cause the onset of acute kidney injury. Unlike chronic kidney disease, however, the kidneys can often recover from acute kidney injury, allowing the patient to resume a normal life. People suffering from acute kidney injury require supportive treatment until their kidneys recover function. They though often remain at increased risk of developing future kidney failure.

The AKI patients which are involved in setting up the sample obtained from control patients with acute kidney injury and the AKI patients on the basis of which the predetermined standard expression level has been obtained are preferably critically ill AKI patients who are treated by intensive care medicine. These critically ill AKI patients usually and preferably developed AKI in the course of sepsis, after a major heart, gastrointestinal (GI) surgery, hypovolemia or major blood loss being characterized by increasing renal retention parameters and/or anuria and/or metabolic acidosis. In those critically ill patients AKI usually develops within 24h after said surgeries or the onset of the sepsis, and AKI is consequently also diagnosed within 24h since the patients are intensively cared. Renal retention parameters, such as urea or creatinine are routinely quantified in the patient's blood sample post surgery. Anuria is the non-passage of urine and usually defined as passage of less than 100 milliliters of urine in a day.

The term "sample" designates a body fluid sample selected from blood, serum, plasma,.

The "patient" or "subject" referred to herein is human. A healthy subject has no signs of disease, weakness, or malfunction, in no particular signs of AKI and no signs of renal malfunction. A patient in accordance with the invention suffers from AKI.

The term "ncRNA" or "non-coding RNA" as used herein designates a functional RNA molecule that is not translated into a protein. The DNA sequence from which a non-coding RNA is transcribed is often called in the art an RNA gene. More than 80% of the human genome is transcribed into RNA transcripts without protein-coding potential [4, 5]. These so called non-coding RNAs (ncRNAs) are arbitrarily separated into long ncRNAs (IncRNAs, ≥ 200 nucleotides) and small ncRNAs (≤200 nucleotides), based on their size. Small RNAs such as microRNAs are currently under intense investigation [6, 7]. However, little is known about the functional role of IncRNAs. LncRNAs play a critical role in several important cellular processes such as the regulation of imprinting and X-chromosome inactivation [8]. Emerging studies in the cancer and cardiovascular field have demonstrated that IncRNAs can be detected in body fluids of patients [9, 10].

As mentioned, the term "IncRNA" or "long non-coding RNA" as commonly used in the art designates an ncRNA comprising more than 200 nucleotides. LncRNAs are generally characterized by nuclear localization, low level of expression and sequence conservation and may be polyadenylated [8]. In the nucleus IncRNAs may "fine-tune" chromatin architecture by interacting with chromatin remodelling complexes (CRCs) to regulate the expression of genes residing on the same chromosome (in cis) or on another chromosome (in trans) [10]. Only a few IncRNAs have thus far been characterized. LncRNAs play a critical role in the regulation of imprinting, exemplified by the long intergenic RNA (lincRNA) Air, and X-chromosome inactivation by Xist (X-inactive specific transcript) [8, 17]. Xist through its co-factor RepA associates with the polycomb complex and thereby initiates epigenetic silencing during X-chromosome inactivation [8, 18]. Air targets repressive histone-modifying activities through molecular interaction with specific chromatin domains to epigenetically silence transcription [8, 19]. The first circulating IncRNA, for which assays with clinical utility have been developed, is prostate cancer gene 3 (PCA3), which was found to specifically diagnose patients with prostate cancer [20, 21]. Indeed, its detection in urine was found to show greater specificity for the detection of prostate cancer than the widely accepted prostate-specific antigen (PSA) test [20, 21].

SEQ ID NO: 1 consists of 498 nucleotides and is therefore a IncRNA. SEQ ID NO: 1 is also designated P5-1104E15.6-001 or TapSAKI ((TrAnscript Predicting Survival in AKI) herein below. Currently, IncRNAs are categorized based mainly on their location with respect to protein-coding genes, since their functional roles are largely unknown. LncRNA can be categorized as: (1) sense or (2) antisense, (3) bidirectional, (4) intronic or (5) intergenic, depending on their position with regard to protein coding genes [8]. The IncRNA of SEQ ID NO: 1 is an intronic antisense IncRNA, transcribed from a gene located in the genome on chromosome 22. A specific role for this IncRNA has not been described yet.

The term "detecting the expression level of ncRNA" means determining the amount or yield of the ncRNA. An ncRNA is initially expressed within a cell. It was found in accordance with the present invention that the ncRNAs of SEQ ID NO: 1 can be detected in the sample of a AKI patient in the sample of a healthy patient, wherein the sample is a blood sample, such as serum or plasma sample. This shows that this IncRNA leaves the cells and is stable outside the cells. An IncRNA being "expressed in a sample" is therefore a IncRNA whose expression level can be detected in the sample by means and methods being further detailed herein below. Hence, the expression level of the ncRNA of SEQ ID NO: 1 can be detected in a sample as required by the methods of the invention. In general, an ncRNA is overexpressed in a test sample if the amount or yield of the ncRNA is greater as compared to the amount or yield of the corresponding ncRNA in a control sample. Likewise, an ncRNA is underexpressed in a test sample if the amount or yield of the ncRNA is less as compared to the amount or yield of the corresponding ncRNA in a control sample. In this context the term "corresponding ncRNA" means that the expression level of the ncRNA of SEQ ID NO: 1 in the test sample is compared to the expression level of the ncRNA of SEQ ID NO: 1 in the control sample.

The methods of the invention rely on the comparison of the expression levels of SEQ ID NO: 1 between a test and a control sample. The expression level in each of the sample to be compared can be quantified by any suitable means and methods available from the art. In general relative and absolute quantification means and methods can be used. In absolute quantification no known standards or controls are needed. The expression level can be directly quantified. As well-known in the art, absolute quantification may rely on a predetermined standard curve. In relative quantification the expression level is quantified relative to a reference (such as known control expressions levels). Also in the absence of controls, one can relatively quantify the expression level when comparing e.g. fluorescence intensities. Suitable means and methods are further detailed herein below.

Methods to assess RNA concentration may, for example, comprise spectrometric methods or measuring the fluorescence intensity of dyes that bind to nucleic acids and selectively fluoresce when bound. Such methods comprise a reverse transcription reaction and the production of cDNA, wherein the amount of the cDNA is determined thereby indirectly determining the amount of the RNA. The fluorescent-based method is particularly useful for cases where the RNA concentration is too low to accurately assess some with spectrophotometry and/or in cases where contaminants absorbing at 260nm make accurate quantification by spectrophotometry difficult or impossible. Methods comprising measuring the fluorescence intensity will be further detailed herein below.

When comparing the expression level of the ncRNA of SEQ ID NO: 1 between different samples reliability of the comparison is preferably improved by including an invariant endogenous control (expression of a reference gene) to correct for potential sample to sample variations. Such normalization with respect to an invariant endogenous control is routinely performed in the art. For example, means and methods for expression level normalization, e.g. in real-time RT-PCR (see, for example, Bustin, Journal of Molecular Endocrinology, (2002) 29, 23-39) or micro-array expression analysis (see, for example, Calza and Balwitan, Methods Mol Biol. 2010;673:37-52) are well-established. Also methods for normalization of the expression levels of small RNA sequences are established (see, for example, Mestdagh et al. (2009) Genome Biol.; 10(6):R64). In case RT-PCR or a micro-array is used to determine the expression levels in accordance with the present invention, the expression levels are preferably normalized to a spiked-in RNA (see, for example, McCormick et al. (2011), Silence, 2:2). Known amounts of a spiked-in RNA are mixed with the sample during preparation. More preferably the RNA is externally spiked-in to plasma and/or serum before the RNA isolation process is carried out, in which case the samples are plasma and/or serum. The spiked-in RNA technology is well-known and commercial kits are available from a number of manufacturers. The spiked-in RNA is preferably a spiked-in C. *elegans* RNA.

In the examples herein below, the levels of circulating IncRNAs were normalized to recombinant *C*. *elegans* miR-39, which was supplemented to the plasma samples during the RNA isolation process. This normalization approach is most preferred in accordance with the present invention.

The test sample and the control sample are preferably obtained from the patients with AKI directly after the patients have been diagnosed as having AKI. In this connection "directly" means with increasing preference within a time frame of 1 week, 5 days, 3 days, 48h, 24h, 12h, 6h and 1h. In this connection the term "about" is preferably ± 15 min.

The methods of the invention refer to (i) the expression level of the ncRNA of SEQ ID NO: 1 in a control blood sample (wherein the control sample may be obtained from AKI patients or a healthy subject) or (ii) a predetermined standard expression level of the ncRNA of SEQ ID NO: 1. In accordance with the first alternative determining the expression level of the ncRNA of SEQ ID NO: 1 in said control sample forms part of the methods of the invention while in accordance with the second alternative the step of determining the expression level of the ncRNA of SEQ ID NO: 1 in the control sample does not form part of the methods of the invention, but has been done before. The resulting predetermined standard expression level of the ncRNA of SEQ ID NO: 1 is then employed in the methods of the invention as a standard of comparison.

To the best knowledge of the inventors the examples herein below show the first evaluation of IncRNAs in a cohort of patients with kidney disease or are the first experimental kidney:IncRNAs study. As can be taken from the examples the study surprisingly revealed: (1) circulating IncRNAs in plasma of critically ill patients with acute kidney injury are detectable, (2) circulating TapSAKI (SEQ ID NO: 1) levels specifically identify patients with AKI, (3) baseline levels of TapSAKI correlated with parameters of disease severity, (4) baseline levels of circulating TapSAKI levels are elevated in non-survivors compared to survivors, (5) TapSAKI was identified as a strong independent prognostic factor for 4 weeks-survival in the multivariate Cox proportional hazards regression analysis and Kaplan-Meier curve analysis. (6) TapSAKI is enriched in hypoxic tubular epithelial cells. Hence, the present study provides insights into levels of circulating IncRNAs in critically ill patients with AKI. A large number of circulating IncRNAs can be reliably detected in plasma of AKI patients. The IncRNA transcript TapSAKI is identified in accordance with the present invention as a novel specific biomarker of AKI, and in particular as a strong and independent predictor of disease severity as well as survival in critically ill patients with AKI.

The present invention relates in a second and related aspect to a method for determining whether a test patient has or is at risk of developing acute kidney injury comprising (a) detecting the expression level of the ncRNA of SEQ ID NO: 1 in a blood sample obtained from said test patient, and (b) comparing said expression level of the ncRNA with the expression level of the ncRNA in a sample obtained from a healthy subject or with a predetermined standard expression level of this ncRNA which was obtained on the basis of at least one sample from a healthy subject, wherein a greater than 3-fold overexpression of the ncRNA in the test patient's sample as compared to the healthy sample or as compared to the predetermined standard is indicative for acute kidney injury.

Again, the method uses isolated samples. Accordingly, the method is in general performed in vitro and preferably does not comprise an invasive step for obtaining the sample from a patient and healthy subject.

A patient determined as having AKI or being at risk of developing AKI preferably receives medical care by medication to treat AKI. On the other hand, a patient determined as having neither AKI nor having a risk of developing AKI preferably receives no medical care by medication to treat AKI.

The method according to the present disclosure may also encompass detecting the expression level of one or more IncRNAs being with increased preference at least 90%, at least 92%, at least 94%, at least 96%, at least 98%, at least 99%, and at least 99.5% identical to SEQ ID NO: 1 independently of or in conjunction with the detection of the expression level of the ncRNA of SEQ ID NO: 1. The method may furthermore encompass detecting the expression level of one or more IncRNAs differing with increasing preference by no more than 10, such as 5, 4, 3, 2 or 1 nucleotide(s) from any one of SEQ ID NO: 1.

Whether the patient has or is at risk of developing AKI can be further assessed by routine diagnosis of the kidney. Non-limiting examples of routine diagnostics are the deterioration of renal function by a measured decrease in urine output. Often AKI is diagnosed on the basis of blood tests for substances normally eliminated by the kidney, in particular urea and creatinine.

As discussed above, circulating levels of SEQ ID NO: 1 were found to specifically identify patients with AKI, and the baseline levels of TapSAKI correlated with parameters of disease severity. It follows that the expression level of the ncRNA of SEQ ID NO: 1 can be used to determining whether a test patient has or is at risk of developing acute kidney injury.

In accordance with a preferred embodiment of the first aspect of the invention, the patient(s) with acute kidney injury is/was treated by renal replacement therapy when or after the sample was obtained.

The patient(s) with acute kidney injury being treated by renal replacement therapy when or after the sample was obtained may be the test patient or the control patients, and are preferably both the test patient and the control patients.

It is to be understood that the sample was obtained in accordance with this preferred embodiment (i) during the time period wherein patient actually received a renal replacement therapy or (ii) in the time frame between two renal replacement therapy interventions, (iii) after the end of the last renal replacement therapy, or (iv) before the patient actually receives the first renal replacement therapy. Within these four options, option (iv) is preferred. Within option (iv) the term "before" means with increasing preference 5 days or less, 4 days or less, 3 days or less, 2 days or less, 1 days or less before the patient actually receives the first renal replacement therapy and on the same day as the patient actually receives the first renal replacement therapy.

The term "renal replacement therapy" (RRT) as used herein designates life-supporting treatments for renal failure, in particular AKI. In AKI a person who likewise had a good response to for example dialysis, and/or who received a kidney transplantation relatively quickly, if indicated and whose body did not reject the transplanted compatible kidney, can very well survive for many years, with relatively good kidney function, before eventually needing intervention again. Early dialysis (and, if indicated, early renal transplantation) in AKI usually brings more favorable outcomes. It may even bring resolution of renal failure in some acute cases. However, it is exceptional for a person to regain total or near-total kidney function. Usually some degree of impairment remains after renal replacement therapy of a AKI patient.

An AKI patient who is RRT dependent is preferably characterized by the loss of kidney function of >30% calculated based on the estimated glomerular filtration rate (eGFR) with either the Modification of Diet in Renal Disease (MDRD) or Cockroft-Gault equation and/or cystatin C-GFR within 48 hours prior to inclusion and oliguria/anuria (<30 mL/h >6 hours prior to inclusion) or hyperkalemia (>6.5 mmol/L) or severe metabolic acidosis (pH<7.15, bicarbonate<12).

In accordance with a more preferred embodiment of the first aspect of the invention, the renal replacement therapy is selected from hemodialysis, peritoneal dialysis, hemofiltration and renal transplantation.

Hemodialysis (also haemodialysis) is a method that is used to achieve the extracorporeal removal of waste products such as creatinine and urea and free water from the blood when the kidneys are in a state of renal failure.

Hemofiltration is similar to hemodialysis and is used almost exclusively in the intensive care setting. Thus, it is almost always used for acute renal failure. It is a *slow continuous* therapy in which sessions usually last between 12 to 24 hours and are usually performed daily. During hemofiltration, a patient's blood is passed through a set of tubing (a filtration circuit) via a machine to a semipermeable membrane (the filter) where waste products and water are removed. Replacement fluid is added and the blood is returned to the patient. The renal replacement therapy is preferred hemodialysis or hemofiltration, and most preferred hemodialysis.

The process of peritoneal dialysis (PD) uses the patient's peritoneum in the abdomen as a membrane across which fluids and dissolved substances (electrolytes, urea, glucose, albumin and other small molecules) are exchanged from the blood. Fluid is introduced through a permanent tube in the abdomen and flushed out either every night while the patient sleeps (automatic peritoneal dialysis) or via regular exchanges throughout the day (continuous ambulatory peritoneal dialysis). PD is used as an alternative to hemodialysis though it is far less commonly used.

Kidney transplantation or renal transplantation is the organ transplant of a kidney usually into a patient with end-stage AKI. Kidney transplantation is typically classified as deceased-donor or living-donor transplantation depending on the source of the donor organ. Living-donor renal transplants are further characterized as genetically related (living-related) or non-related (living-unrelated) transplants, depending on whether a biological relationship exists between the donor and recipient.

In accordance with a preferred embodiment of the first aspect of the invention, the at least greater than 1.5-fold over- and underexpression as compared to the control patient's sample is at least greater than 2-fold over- and underexpression, preferably at least 2.5-fold over- and underexpression, and most preferably least 3-fold over- and underexpression.

In accordance with another preferred embodiment of the first aspect of the invention, the the at least greater than 5-fold over- and underexpression as compared to the healthy subject is at least greater than 10-fold over- and underexpression, preferably at least 20-fold over- and underexpression, and most preferably least 30-fold over- and underexpression.

In accordance with a preferred embodiment of the second aspect of the invention, the at least greater than 3-fold over- and underexpression as compared to the sample of the healthy subject is at least greater than 5-fold over- and underexpression, preferably at least 10-fold over- and underexpression, and most preferably least 15-fold over- and underexpression.

In accordance with the method of the first aspect of the invention a 1.5-fold under- and/or overexpression, respectively, of the IncRNA of SEQ ID NO: 1 as compared to the respective control patient's sample is of sufficient value for predicting mortality of a test patient with acute kidney injury, and/or a 5-fold overexpression of the IncRNA of SEQ ID NO: 1 as compared to the sample of the of the healthy subject is of sufficient value for predicting mortality of a test patient with acute kidney injury. Similarly in accordance with the method of the second aspect of the invention a 3-fold overexpression of the IncRNA of SEQ ID NO: 1 as compared to the sample of the of the healthy subject is of sufficient value for determining whether a test patient has or is at risk of developing acute kidney injury. However, it is also known that the confidence level of a diagnostic method may be increased by increasing the expression level threshold which is assumed to be indicative for a certain event. For this reason, the greater under- and/or overexpression threshold values according to the above embodiments of the method of the invention are preferred.

In this respect it is of note that for the expression levels of the IncRNA of SEQ ID NO: 1 an expression gradient was found among the tested subjects, wherein in the samples of healthy subjects consistently the lowest expression levels were found, in the samples of AKI patients which survived for a long term significantly higher expression levels as in healthy subjects were found, and in the samples of AKI patients which died within four weeks on renal failure significantly higher expression levels as in AKI patients which survived for a long term as well as in healthy subjects were found.

In accordance with a further preferred embodiment of the first and second aspect of the invention, the detection of the expression level of the ncRNA comprises (i) quantitative PCR, preferably quantitative real time PCR, or (ii) a template/RNA amplification method followed by determining the expression level of the ncRNA using a fluorescence- or luminescence-based quantification method.

In quantitative PCR (qPCR), the amount of amplified product is linked to fluorescence intensity using a fluorescent reporter molecule. The point at which the fluorescent signal is measured in order to calculate the initial template quantity can either be at the end of the reaction (endpoint semi-quantitative PCR) or while the amplification is still progressing (real-time qPCR).

In endpoint semi-quantitative PCR, fluorescence data are collected after the amplification reaction has been completed, usually after 30-40 cycles, and this final fluorescence is used to back-calculate the amount of template present prior to initiating PCR.

The more sensitive and reproducible method of real-time qPCR measures the fluorescence at each cycle as the amplification progresses. This allows quantification of the template to be based on the fluorescence signal during the exponential phase of amplification, before limiting reagents, accumulation of inhibitors, or inactivation of the polymerase have started to have an effect on the efficiency of amplification. Fluorescence readings at these earlier cycles of the reaction will measure the amplified template quantity where the reaction is much more reproducible from sample to sample than at the endpoint.

A non-limiting example of a template/RNA amplification method followed by determining the expression level of the one or more IncRNAs using a fluorescence- or luminescence-based quantification method is a method combining transcription mediated amplification (TMA) and a hybridization protection assay (HPA). In more detail, such method may comprise hybridizing an oligonucleotide ("capture oligonucleotide") that are complementary to SEQ ID NO: 1. The hybridized target sequences are then captured onto magnetic microparticles that are separated from the sample in a magnetic field. Wash steps may be utilized to remove extraneous components. Target amplification typically occurs via TMA, which is a transcription-based nucleic acid amplification method that utilizes two enzymes, Moloney murine leukemia virus (MMLV) reverse transcriptase and T7 RNA polymerase. A unique set of primers is used for to target SEQ ID NO: 1. The reverse transcriptase is used to generate a DNA copy (containing a promoter sequence for T7 RNA polymerase) of the target sequence. T7 RNA polymerase produces multiple copies of RNA amplicon from the DNA copy. Detection of IncRNA expression level is achieved by HPA using single-stranded, chemiluminescent-labeled nucleic acid probes that are complementary to the one or more amplicon. Preferably, distinguishably labelled probes are used for each target amplicon. The labeled nucleic acid probes hybridize specifically to the amplicon. A "selection reagent" then differentiates between hybridized and unhybridized probes by inactivating the label on unhybridized probes. During the detection step, the chemiluminescent signal produced by the hybridized probe is measured in a luminometer and is reported as "Relative Light Units" (RLU), thereby quantifying the IncRNA expression level.

In accordance with a more preferred embodiment of the first and second aspect of the invention, the primer sequences of SEQ ID NOs 2 and 3 are employed for the detection of the expression level of SEQ ID NO: 1

The primer sequences of SEQ ID NOs 2 (forward primer) and 3 (reverse primer) are employed for the detection of the expression level of SEQ ID NO: 1 in the examples of the application and have proven to be especially useful and reliable.

In accordance with invention, the samples are blood and preferably plasma samples.

The term "blood sample" encompasses whole blood as well as any blood-derived sample, in particular plasma or serum. Most preferably the blood sample is a plasma sample.

In accordance with another preferred embodiment of the first and second aspect of the invention, the method comprises prior to the detection of the expression level of the ncRNA a pre-amplification step of the RNA within the test patient's sample, the control patients' sample and/or the sample from a healthy subject.

Performing a pre-amplification step is of particular advantage in case only low amount of (test and/or control) sample is available. The pre-amplification step allows increasing the amount of RNA within the sample before proceeding to the analysis of the expression level. Means and methods for the pre-amplification of RNA are well known in the art (see, e.g., Vermeulen et al (2009) BMC Res Notes., 2:235). In case both the RNA in the test and control sample is pre-amplified preferably the same method for the pre-amplification step is used such that the relative amount of RNA in the test sample as compared to the control sample is maintained. In case only the RNA of the test or control sample is pre-amplified or the two RNA samples are pre-amplified by different methods, the expression level data may have to be normalized for the pre-amplification step; see, e.g. Mestdagh et al. (2009), Genome Biology 2009, 10: R64.

In accordance with a further preferred embodiment of the first and second aspect of the invention, the test patient and the control patient(s)/healthy subject are matched by one or more of age, sex, diabetes mellitus, renal failure etiology, and race.

Matching the test patient and the control patient(s)/healthy subject (noting that depending on the actual embodiment of method of the invention either control patient(s) and/or a healthy subject are/is employed) by one or more of age, sex, diabetes mellitus, renal failure etiology, and race will additionally increase the reliability of the methods of the invention, since any potential expression level differences caused by differences in age, sex, diabetes mellitus state, renal failure etiology, and/or race can be essentially excluded. As can be taken from the examples the healthy control subjects were age-matched with the AKI patients. Hence, matching by age is preferred in accordance with this embodiment.

In accordance with a still further preferred embodiment of the first and second aspect of the invention, the control patients/healthy subject are/is at least 3 control patients/healthy subjects, preferably a least 5 control patients/healthy subjects, and more preferably a least 10 control patients/healthy subjects.

In more detail, the control within the methods of the invention is either based on AKI patients which were alive or dead at least about 4 weeks after diagnosis of the acute kidney injury, and/or based on a healthy subject. Increasing the number of control patients/healthy subjects to at least 3 patients, preferably a least 5 patients, and more preferably a least 10 control patient/healthy subjects is expected to additionally increase the reliability of the methods of the invention, because potential expression level abnormalities in a given control patient/healthy subject are normalized by the other control patients. This holds true irrespective of whether the methods of the invention refer to (i) the expression level of the ncRNA of SEQ ID NO: 1 in a (control) sample or (ii) a predetermined standard expression level of the ncRNA of SEQ ID NO: 1. Also even higher numbers of control patients/healthy subjects may be employed, such as 25, 50 or 100 control patients/healthy subjects.

In a third and related aspect the present invention relates to a kit for predicting mortality of a test patient with acute kidney injury and/or for determining whether a test patient has or is at risk of developing acute kidney injury, said kit comprising means for the detection of the expression level of the ncRNA of SEQ ID NO: 1 and instructions how to use the kit, wherein the means for the detection of the expression level of the ncRNA of SEQ ID NO: 1 comprise the primer pair of SEQ ID NOs 2 and 3. The various components of the kit may be packaged in one or more containers such as one or more vials. The vials may, in addition to the components, comprise preservatives or buffers for storage. In addition, the kit will contain instructions for use.

The instructions how to use the kit comprise a leaflet explaining how the ncRNA of SEQ ID NO: 1 is to be used for predicting mortality of a test patient with acute kidney injury and/or for determining whether a test patient has or is at risk of developing acute kidney injury. Hence, the kit comprises a leaflet explaining how to carry out of the method of the fist and/or second aspect of the invention.

The kit preferably further comprises (i) a sample obtained from control patients with acute kidney injury, wherein the control patients were either all alive or all dead at least about 4 weeks after diagnosis of the acute kidney injury, and/or a sample obtained from a healthy subject, or (ii) a predetermined standard expression level of the ncRNA of SEQ ID NO: 1 obtained from one or more of said samples.

Techniques for designing specific primers are well known in the art (see, for example, http://www.thermoscientificbio.com/uploadedFiles/Resources/guidelines-primer-design.pdf). In the kit of the present invention, the primer pairs used for the specific detection of the expression level of the ncRNA of SEQ ID NO: 1 comprise the primer pair of SEQ ID NOs 2 and 3.

The figures show:
- **Fig 1:**: Hierarchical cluster (A) and volcano plot analysis (B) of deregulated IncRNAs in plasma of patients with AKI and healthy controls.
- **Fig 2:**: Validation of significantly deregulated transcripts in agarose gel electrophoresis in kidney biopsies (A) as well as plasma of AKI patients (B). Bp = base pairs; H20 = water control; L263-L282 = primer numbers

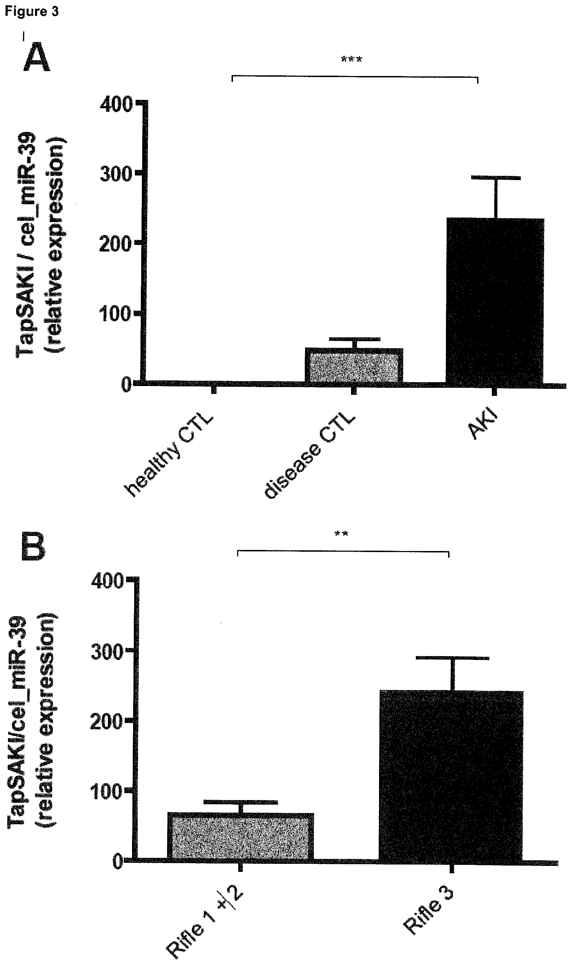

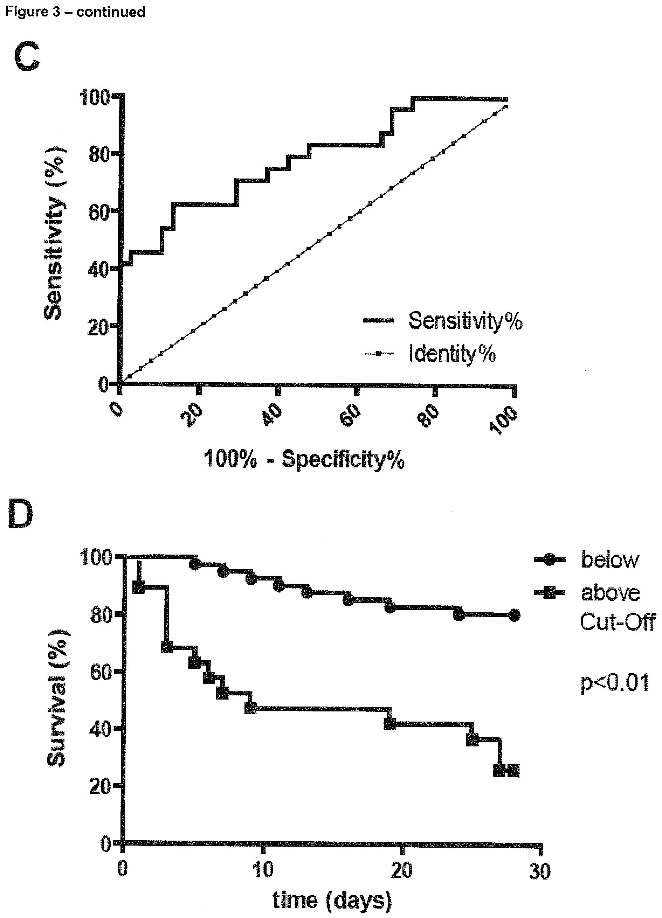

- **Fig 3:**: Levels of circulating TapSAKI are strongly deregulated in patients with acute kidney injury (AKI) vs. AMI patients and healthy controls (CTL) (p<0.0001) (A). Baseline levels of circulating TapSAKI were significantly lower in RIFLE stadium 1 and 2 compared to stadium 3 (p<0.01) (B). ROC curve analysis (C) in critically ill patients with acute kidney injury identifies a cut point of 133 relative expression with an area under the curve (AUC) of 0.8, a sensitivity of 63% and specificity of 87%. Kaplan-Meier curve of survival (D) in critically ill patients with acute kidney injury stratified to circulating TapSAKI above and below a cut point of 133 relative expression. Log rank test confirmed statistical significance for TapSAKI (p<0.01).
- **Fig 4:**: Algorithm to detect and validate circulating IncRNAs in plasma of patients.

The examples illustrate the invention.

### Example 1 - Materials and Methods

The example section of the present application is a post-hoc measurement of prospectively collected blood samples from the HANDOUT trial [11]. Patients in seven ICUs of the tertiary care centre at the Hannover Medical School suffering from AKI were evaluated for inclusion. The study protocol was approved by the Hannover Medical School Ethics Committee and was conducted in accordance with the declaration of Helsinki and German Federal Guidelines. Patients in seven ICUs of the tertiary care centre at the Hannover Medical School suffering from AKI were evaluated for inclusion. The inclusion criteria were non-post-renal AKI with RRT dependence indicated by the loss of kidney function of >30% calculated estimated glomerular filtration rate (eGFR) with either the Modification of Diet in Renal Disease (MDRD) or Cockroft-Gault equation and/or cystatin C-GFR within 48 hours prior to inclusion and oliguria/anuria (<30 mL/h >6 hours prior to inclusion) or hyperkalemia (>6.5 mmol/L) or severe metabolic acidosis (pH<7.15, bicarbonate<12). Exclusion criteria were pre-existing chronic kidney disease as defined by eGFR <60mL/min or a serum creatinine concentration >1.7 mg/dL more than 10 days prior to initiation of the first RRT. Additionally, the presence of AV-fistula or dialysis catheter as indicative of chronic kidney disease was considered. Further exclusion criteria were participation in another study, consent denial or withdrawal and need for extracorporeal membrane oxygenation therapy. The enrolment was performed by attending nephrologists after obtaining written informed consent from a patient or his/her legal representatives. If the patient was recovering and able to communicate, he/she was informed of the study purpose and consent was required to further maintain his/her status as a study participant.

After inclusion, the specific medical condition leading to RRT initiation was documented out of a list of four possible causes requiring immediate RRT. All patients received a nutritional intake of at least 25-30 kcal/kg/day, preferentially delivered as enteral nutrition. The prescribed protein intake was >1.2 g/kg/day. RRT in all patients was performed in a slow extended dialysis mode using the GENIUSTM dialysis system (Fresenius Medical Care, Bad Homburg, Germany) as described in detail elsewhere [12]. The dose of the RRT was tailored according to the patient individual need, starting with at least one treatment daily. RRT was discontinued in patients meeting the following criteria for renal recovery: urine output >1000 mL/day and/or increased solute clearance, i.e. decline in pre-treatment serum creatinine concentration with eGFR >15 mL/min (by either MDRD, Cockroft-Gault equation and/or cystatin C-GFR). Blood was drawn immediately before the start of RRT. Serum creatinine and serum C reactive protein (CRP) levels were determined by routine laboratory methods. Sequential Organ Failure Assessment (SOFA) score [13] and Acute Physiology and Chronic Health Evaluation II (APACHE II) score [14] were obtained for each patient immediately before initiation of RRT. The presence of sepsis was defined according to the SCCM/ESICM/ACCP/ATS/SIS International Sepsis Definitions [15]. AKI was classified post- HOC by means of the RIFLE criteria at initiation of RRT [16].

### Study outcomes and statistical analysis

The main objective of the study was to analyze the predictive value of circulating IncRNAs concerning mortality and renal recovery of critically ill patients with AKI receiving RRT. The study endpoint was defined as survival 4 weeks after initiation of RRT and renal recovery (no RRT requirement) in survivors 4 weeks after initiation of RRT.

### RNA Isolation and IncRNA transcriptome analysis

Plasma samples were obtained before initiation of RRT and immediately stored at -80°C. Plasma samples were centrifuged before storage at -80°C and immediately after thawing and before RNA isolation. Total RNA, including small RNAs, was isolated from 100µl of plasma using miRNeasy mini columns (Qiagen, Hilden, Germany) according to the manufacturer's instructions. As an internal spike-in control, Caenorhabditis elegans cel-miR-39 was added during the isolation process. RNA quantity and quality were measured by NanoDrop ND-1000 and RNA integrity was assessed by standard denaturing agarose gel electrophoresis. The remarkable stability of IncRNAs in plasma after repetitive freeze/thaw cycles and subjection to room temperature for up to 24 hours was previously demonstrated [9]. LncRNA transcriptome analysis was performed by using microarray-based global transcriptome analysis (Arraystar Human LncRNA array; version 2.0), which allow simultaneous detection of 33,045 IncRNAs. To define a potential good biomarker candidate IncRNA, the following strategy was chosen: all IncRNA transcripts that were included in the microarrays according to their average signal intensity were firstly sorted. The signal intensity for each IncRNA transcript ranged between five (lowest value) to 17 (highest value). For Raw LncRNA data normalization and low intensity filtering raw signal intensities were normalized using the quantile method by GeneSpring GX v11.5.1, and low intensity LncRNAs were filtered.

### Microarray processing

Arraystar Human LncRNA Microarray V3.0 is designed for the global profiling of human LncRNAs and protein-coding transcripts, which is updated from the previous Microarray V2.0. About 30,586 LncRNAs and 26,109 coding transcripts can be detected by this third-generation LncRNA microarray.

### RNA labeling and array hybridization

Sample labeling and array hybridization were performed according to the Agilent One-Color Microarray-Based Gene Expression Analysis protocol (Agilent Technology) with minor modifications. Briefly, mRNA was purified from total RNA after removal of rRNA (mRNA-ONLY™ Eukaryotic mRNA Isolation Kit, Epicentre). Then, each sample was amplified and transcribed into fluorescent cRNA along the entire length of the transcripts without 3' bias utilizing a random priming method. The labeled cRNAs were purified by RNeasy Mini Kit (Qiagen). The concentration and specific activity of the labeled cRNAs (pmol Cy3/µg cRNA) were measured by NanoDrop ND-1000. One µg of each labeled cRNA was fragmented by adding 5 µl 10 × Blocking Agent and 1 µl of 25 × Fragmentation Buffer, then the mixture was heated at 60 °C for 30 min, finally 25 µl 2 × GE Hybridization buffer was added to dilute the labeled cRNA. 50 µl of hybridization solution was dispensed into the gasket slide and assembled to the LncRNA expression microarray slide. The slides were incubated for 17 hours at 65°C in an Agilent Hybridization Oven. The hybridized arrays were washed, fixed and scanned by using the Agilent DNA Microarray Scanner (part number G2505C).

### Microarray data analysis

Agilent Feature Extraction software (version 11.0.1.1) was used to analyze acquired array images. Quantile normalization and subsequent data processing were performed by using the GeneSpring GX v11.5.1 software package (Agilent Technologies). After quantile normalization of the raw data, LncRNAs and mRNAs with flags in at least 3 out of 9 samples as at least Present or Marginal ("All Targets Value") were chosen for further data analysis. Differentially expressed LncRNAs and mRNAs with statistical significance were identified through Volcano Plot filtering between two groups of samples. p-values were calculated by t-test. Hierarchical Clustering was performed using the Agilent GeneSpring GX software (version 11.5.1).

### LncRNA validation

LncRNA sequences were assessed using the ENSEMBL genome browser (ensembl.org) and their detection was next validated by conventional polymerase chain reaction (PCR) and agarose gel electrophoresis. For this purpose, the isolated RNA was reverse transcribed with random primers (iScript™ Select cDNA Synthesis Kit; Bio-Rad, Germany) using RNA isolated from human kidney biopsies and plasma of AKI patients as well as controls. Amount of RNA used for reverse transcription: 100 ng for plasma and 500 ng for kidney biopsies. Specific IncRNAs were finally amplified by real-time PCR analysis (iQ™ SYBR® Green Supermix #170-8880, Bio-Rad, Germany) in RNA isolated from plasma of patients using primers listed in the following Table 1.

**Table 1: Oligonucleotide sequences used for IncRNA detection in plasma (TapSAKI depicted in red)**

| LncRNA | Forward primer | Reverse primer |
|---|---|---|
| ASHGA5P044509 | AGGAAGGTGACCATGAGAGA | TGCACTGAACAAGAAAGGCT |
| | | |
| ASHGA5P034969 | GGGTCCAACAGTGAAGAACG | TGTTAACTTTGTGGCAGGCA |
| | | |
| ASHGA5P037701 (TapSAKI) | GTAGACCATGACACCCAGCA | CTCGTCTCTGCTCCATGGTGA |

| | | |
|---|---|---|
| C.elegans-miR-39 was amplified using a TaqMan assay as a normalization control (Applied Biosystems, U.S.A.). Relative expression was analyzed by ΔΔCt analysis. LncRNA levels were validated in 109 AKI patients, 30 disease controls and 30 age-matched healthy controls (16 males, 14 females; age: 54 (46-66)). The TapSAKI sequence in plasma of AKI patients as well as controls was confirmed by sequencing analysis. | | |

### Statistical analysis

Continuous variables are expressed as medians with corresponding 25th and 75th percentiles (IQR) and were compared by using the Mann-Whitney rank sum test or the Kruskal Wallis one-way analysis of variance. Categorical variables were compared using the X2 test. Variables were assessed for normal distribution using the Kolmogorov-Smirnov test. Correlations between variables were assessed by the Spearman rank correlation coefficient. Parameters independently associated with survival were identified by univariate and multivariate Cox proportional hazards models. Variables found to be statistically significant at a 10% level in the univariate analysis were included in the multivariate model using backward elimination. Two-sided p-values <0.05 were considered statistically significant for all statistical procedures used. The distribution of the time-to-event variables were estimated using the Kaplan-Meier method with log-rank testing. For comparison of the prognostic values of IncRNAs receiver operating characteristic (ROC) curves were generated and the areas under the curves (AUCs) were calculated. All statistical analyses were performed with the SPSS package (SPSS Inc., Chicago, IL, USA) and GraphPad Prism software (GraphPad Prism Software Inc. San Diego, California, USA).

### Example 2 - Results

### LncRNA expression analysis in plasma

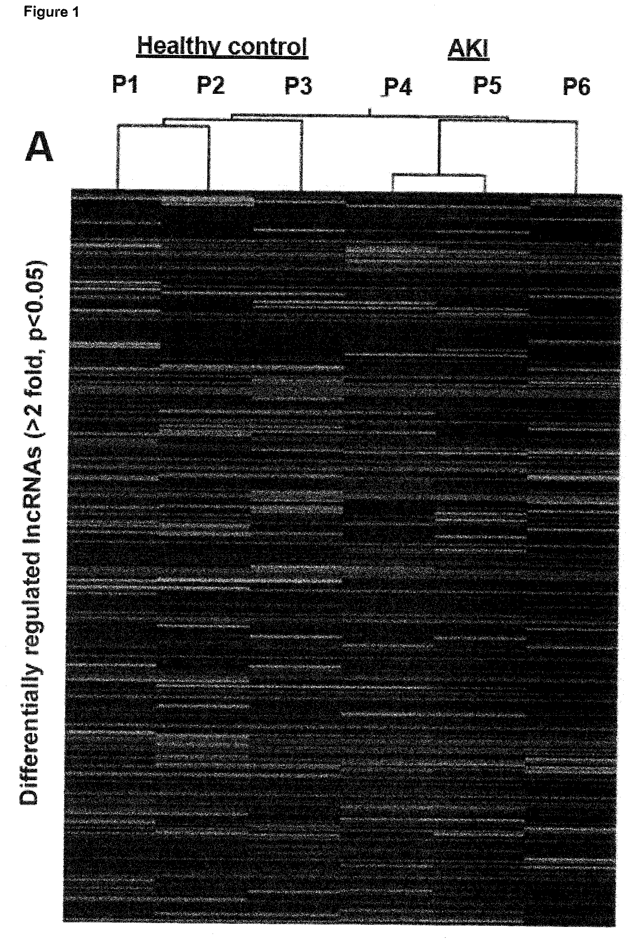

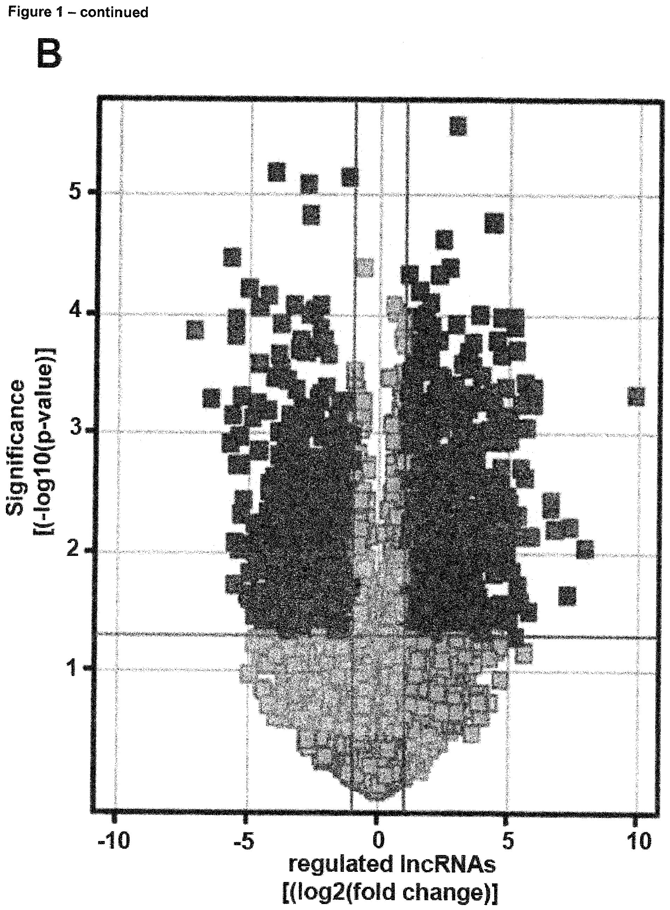

To assess an impact of AKI on circulating IncRNAs, a genome-wide expression analysis in RNA isolated from plasma of patients with AKI at inception of RRT (n=3) and healthy age-matched controls (n=3) was conducted. Figure 1 shows that hierarchical clustering analysis (A) and volcano plot analysis (B) clearly distinguishes AKI patients from controls as evidenced by a specific signature of significantly regulated circulating IncRNAs. The clinical characteristics of the whole cohort of AKI patients (n=109) are shown in Table 2.

| Table 2 - Demographic, clinical and laboratory characteristics of patients | | | | |
|---|---|---|---|---|
| | Total | survivors | non-survivors | p-value |
| Number of patients | 109 | 69 | 40 | 0.64 |
| Male (n; %) | 64 (59) | 40 | 25 | |
| Female (n; %) | 45 (41) | 29 | 15 | |
| | | | | |
| Discipline of ICU admission | | | | 0.39 |
| Medicine (n; %) | 46 (42) | 27 (39) | 19 (48) | |
| General surgery (n; %) | 27 (25) | 16 (23) | 11 (28) | |
| Cardiac surgery (n; %) | 36 (33) | 26 (38) | 10 (24) | |
| Age (years) | 52 (40-63) | 52 (44-63) | 51 (37-63) | 0.77 |
| BMI (kg/m²) | 25 (22-28) | 25.4 (22-28) | 24.7 (22-28) | 0.62 |
| Indication for RRT | | | | |
| eGFR loss >30 % | 93 | 59 | 34 | 0.94 |
| Oliguria/anuria | 71 | 46 | 25 | 0.77 |
| Metabolic acidosis | 8 | 4 | 4 | 0.42 |
| Hyperkalemia | 6 | 2 | 4 | 0.12 |
| SOFA score | 13 (10-15) | 14 (11-15.5) | 13 (10-15) | 0.68 |
| Renal | 2 (1.5-3) | 2 (1-2.5) | 2 (2-3) | 0.02* |
| Coagulation | 1 (0-2) | 2 (0-2) | 1 (0-2) | 0.62 |
| Cardiovascular | 4 (0.5-4) | 4 (2-4) | 3 (0-4) | 0.23 |
| Nervous system | 4 (3-4) | 4 (3-4) | 4 (3-4) | 0.94 |
| Respiratory system | 2 (1-3) | 2 (1-3) | 2 (2-3) | 0.83 |
| Liver | 2 (0-2) | 2 (0-3) | 2 (0-2) | 0.39 |
| RIFLE class | | | | 0.13 |
| Risk (n; %) | 10 (9) | 9 (13) | 1 (3) | 0.07 |
| Injury (n; %) | 15 (14) | 9 (13) | 6 (15) | 0.8 |
| Failure (n; %) | 84 (77) | 51 (74) | 33(82) | 0.3 |
| | | | | |
| APACHE II score | 34 (27-36) | 33 (26-36) | 35 (29-39.8) | 0.58 |
| CRP (mg/L) | 113 (56-197) | 82 (46-191) | 145.5 (67-211.5) | 0.16 |
| MAP (mmHg) | 75.5 (67-90) | 77 (70-93) | 74 (64-86) | 0.27 |
| Heart rate (bpm) | 100 (85-110) | 99 (84-109) | 100 (89-111) | 0.27 |

| | | | | |
|---|---|---|---|---|
| ICU = intensive care unit; BMI = body mass index; RRT = renal replacement therapy; eGFR = estimated glomerular filtration rate; SOFA = Sequential Organ Failure Assessment; APACHE II = Acute Physiology and Chronic Health Evaluation II; MAP = mean arterial blood pressure; CRP = C-reactive protein; n = number of patients. | | | | |

The levels of 4.410 circulating IncRNAs were detectable in all groups of the array cohort, showed a signal intensity >9 and differed between patients and healthy controls. The levels of 20 IncRNAs, which were up-regulated > 5-fold were included for further analysis; see table 3:

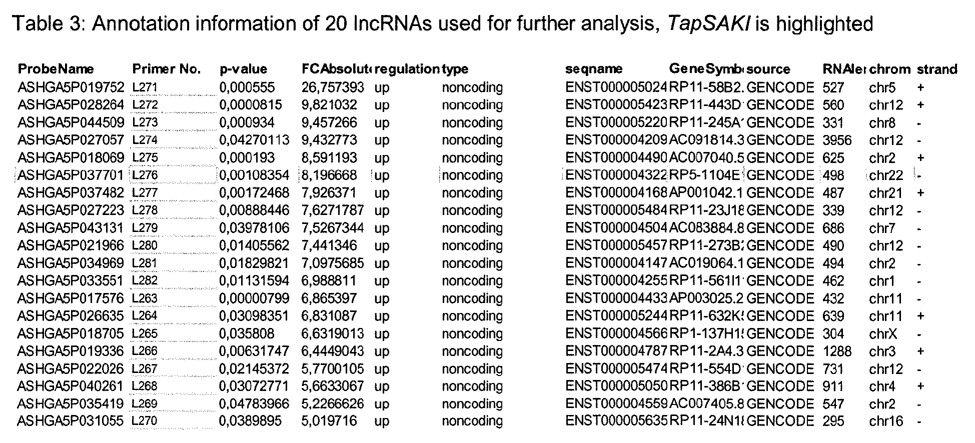

### LncRNA validation in tissue and plasma

The detection and amplification of IncRNAs in plasma is challenging due to their low abundance. In order to investigate the detectability of deregulated circulating IncRNAs transcripts were first amplified by using RNA isolated from renal biopsy specimens of patients (n=6) by conventional polymerase chain reaction (PCR) and separated the transcripts by agarose gel electrophoresis. Patient characteristics of patients included for kidney biopsy are shown in table 4.

| Table 4 - Characteristics of patients included for kidney biopsy | | | | | | |
|---|---|---|---|---|---|---|
| Patient No. | 1 | 2 | 3 | 4 | 5 | 6 |
| Age (years) | 44 | 69 | 34 | 77 | 51 | 57 |
| Gender (m/f) | m | f | m | f | f | f |
| Native (n)/ transplant kidney (t) | n | n | n | t | t | t |
| RIFLE stadium | Failure | Injury | Injury | Injury | Failure | Injury |
| Histological | drug | drug | RPGN | rejection | interstitial | hypovolemia |
| diagnosis / cause of AKI | toxicity | toxicity | | | nephritis | |

| | | | | | | |
|---|---|---|---|---|---|---|
| RPGN = rapid progressive glomerulonephritis | | | | | | |

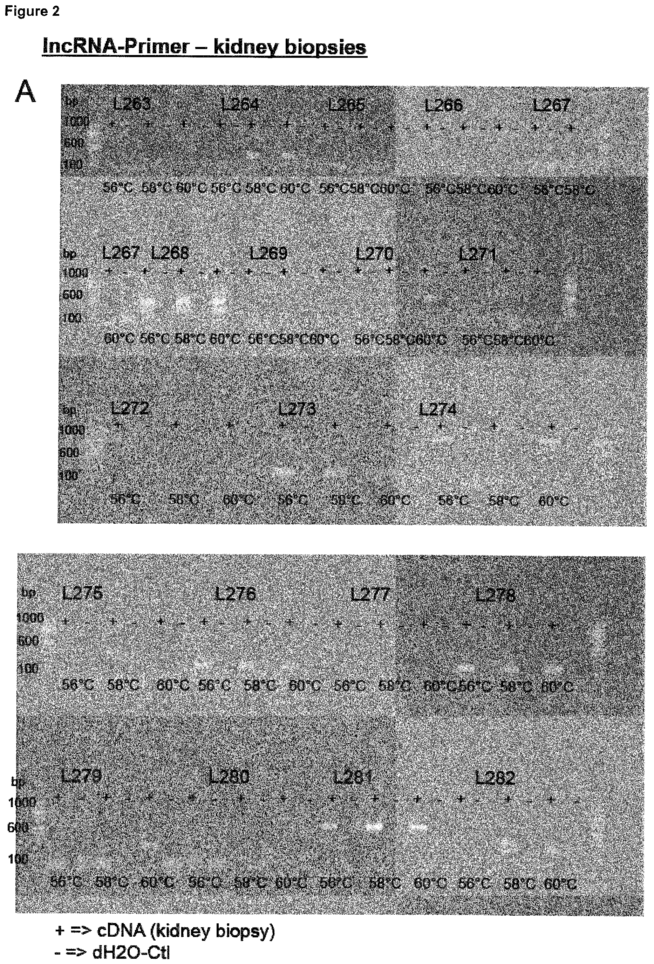

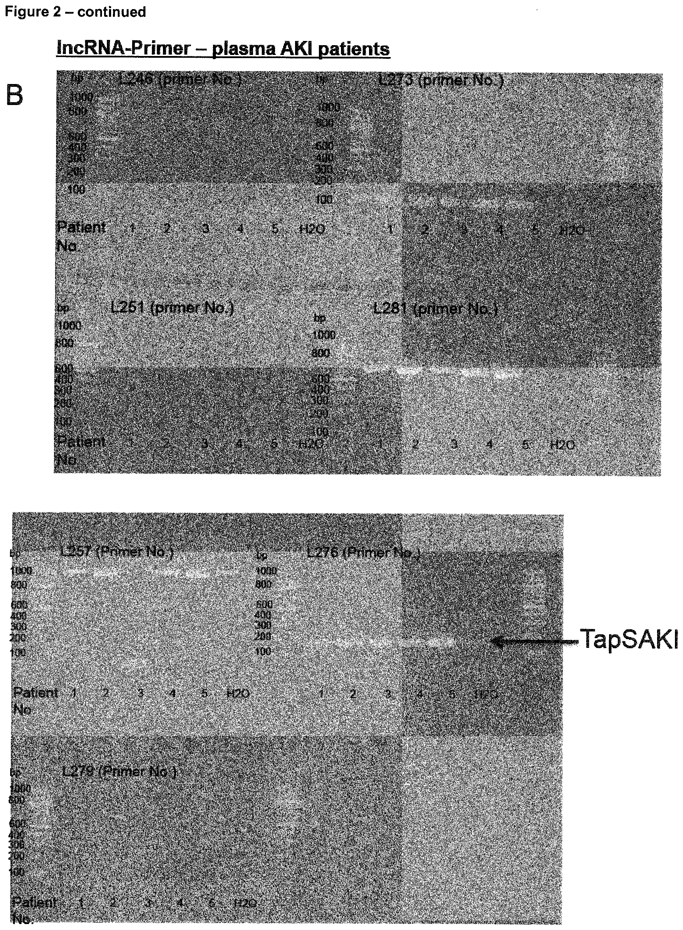

Ten transcripts were found to show specific bands of correct size (see Figure 2A and B). Next, these transcripts were tested in RNA isolated from plasma of patients with AKI and found 3 transcripts to show specific bands (see Figure 2C). Real-time PCR analysis was then performed in a subset of patients with AKI (n=10). The novel transcript RP5-1104E15.6-001 (ensembl.org) showed clean amplification curves, specific curves in melting curve analysis and were undetectable in water controls without template. Primer sequences of tested IncRNAs and annotation information are given in table 3. RP5-1104E15.6-001 is an intronic IncRNA and originates from the gene RP5-1104E15.6 (ensembl.org), which is located on chromosome 22. The transcript runs antisense to beta-1,4-mannosyl-glycoprotein 4-beta-N-acetylglucosaminyltransferase (MGAT3). For the sake of clarity RP5-1104E15.6-001 is also referred to as TapSAKI ((TrAnscript Predicting Survival in AKI). In order to ascertain that TapSAKI was specifically detected in plasma, transcripts were sequenced in these patients after PCR amplification, which confirmed that TapSAKI was correctly amplified and detectable in plasma.

### TapSAKI is a biomarker of AKI

TapSAKI in the whole cohort of patients with AKI (n=109), AMI (n=30) and age-matched healthy individuals (n=30) was next investigated. As shown in Figure 3A, TapSAKI was significantly elevated compared to healthy controls and disease controls, underlining the specificity as a biomarker in AKI (p<0.0001). Baseline TapSAKI levels were elevated in patients with RIFLE stadium 3 as compared to RIFLE stadium 1 and 2 (p<0.01; see Figure 3B). Baseline TapSAKI levels correlated with bicarbonate levels (r=0.3, p=0.02), leukocyte count (r=0.4, p<0.01), length of need for respirator therapy (r=-0.3, p=0.03) and length of stay on the intensive care unit (r=-0.4, p<0.01).

### TapSAKI predicts survival in AKI

Patients were grouped as survivors and non-survivors at 4 weeks after initiation of RRT. Patient groups were comparable with respect to baseline demographics and the proportion of RIFLE categories (Table 5). A total of 40 patients died in the cohort. Levels of circulating TapSAKI were significantly (p<0.05) elevated in non-survivors. In order to investigate the prognostic potential of circulating TapSAKI levels at initiation of RRT with regard to overall mortality, univariate Cox proportional hazards analyses was performed. In the cohort of 109 critically ill patients with AKI TapSAKI levels, major surgery, sepsis, shock, APACHE II and SOFA score showed prognostic significance at a 10% level and were subsequently subjected to multivariate Cox regression analysis; see Table 5.

**Table 5 - Univariate and multivariate Cox regression analysis for survival**

| Univariate | Multivariate | | | | | |
|---|---|---|---|---|---|---|
| Variables | HR | 95% Cl | p-value | HR | 95% Cl | p-value |
| TapSAKI (relative expression) | 1.868 | 1.079 to 3.232 | 0.008* | 1.719 | 0.999 to 2.959 | 0.006* |
| Age (years) | 0.996 | 0.975 to 1.016 | 0.7 | | | |
| Gender (m/f) | 0.839 | 0.442 to 1.592 | 0.591 | | | |
| Body mass index | 0.996 | 0.935 to 1.061 | 0.896 | | | |
| Sepsis (yes/no) | 2.222 | 1.194 to 4.134 | 0.012* | | | n.s. |
| Diabetes (yes/no) | 1.137 | 0.445 to 2.903 | 0.788 | | | |
| Surgery (yes/no) | 1.995 | 1.040 to 3.828 | 0.04* | | | n.s. |
| Shock (yes/no) | 2.618 | 0.928 to 7.382 | 0.069* | | | n.s. |
| S-creatinine | 0.998 | 0.987 to 1.010 | 0.801 | | | |
| RIFLE criteria | 1.578 | 0.855 to 2.912 | 0.145 | | | |
| SOFA score | 1.141 | 1.039 to 1.253 | 0.006* | | | |
| APACHE II score | 1.040 | 0.999 to 1.083 | 0.05* | 1.063 | 1.017 to 1.111 | 0.007* |
| CRP | 1.001 | 0.998 to 1.003 | 0.516 | | | |
| Heart rate | 1.003 | 0.989 to 1.016 | 0.699 | | | |

| | | | | | | |
|---|---|---|---|---|---|---|
| APACHE II = Acute Physiology and Chronic Health Evaluation II; Cl = confidence interval; HR = hazard ratio; TapSAKI = Transcript predicting Survival in AKI (IncRNA); n.s. = non-significant; SOFA = Sequential Organ Failure Assessment; *=p<0.1. | | | | | | |

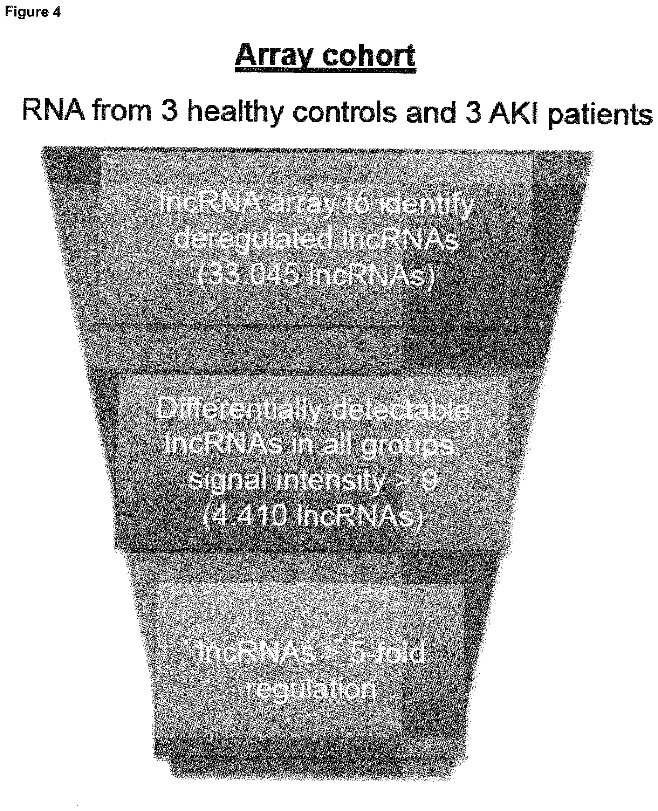

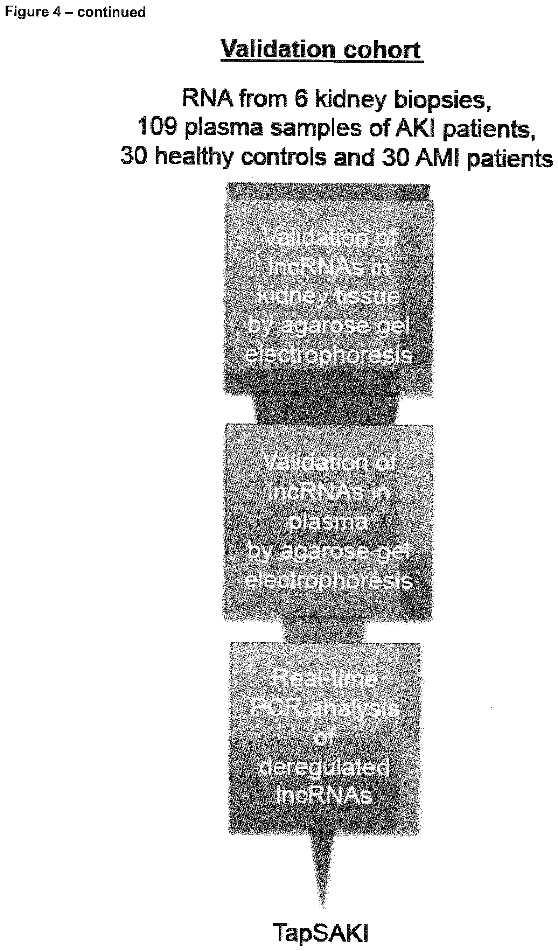

Only APACHE II score (p=0.007) and TapSAKI levels (p=0.006) remained independent predictors of survival in multivariate analysis. In ROC curve analysis TapSAKI levels yielded an area under the ROC curve (AUC) value of 0.8 (standard error of the mean: 0.06; 95% confidence interval: 0.67 to 0.91; p<0.0001). In ROC curve analysis a cut point with 63% sensitivity and 87% specificity was defined (Figure 3C, a level of 133 relative expression by ΔΔCt analysis). Figure 3D illustrates the Kaplan-Meier curve of survival 4 weeks after initiation of RRT stratified to TapSAKI levels above and below the identified cut point. Log rank test confirmed statistical significance for circulating TapSAKI levels (p<0.01). The used detection algorithm for circulating IncRNAs is given in Figure 4.

### TapSAKI is specifically enriched in hypoxic renal tubular epithelial cells

In order to confirm the potential origin of TapSAKI its regulation in hypoxic renal endothelial and tubular epithelial cells was assessed and TapSAKI was found to be specifically enriched in cultured tubular epithelial cells (HK-2) subjected to ATP depletion (p<0.05, data not shown).

### References

1. Barrantes F, Tian J, Vazquez R, Amoateng-Adjepong Y, Manthous CA: Acute kidney injury criteria predict outcomes of critically ill patients. Crit Care Med 2008; 36: 1397-1403.
2. Mehta RL, Kellum JA, Shah SV, Molitoris BA, Ronco C, Warnock DG, et al.; Acute Kidney Injury Network (AKIN): report of an initiative to improve outcomes in acute kidney injury. Crit Care 2007;11: R31.
3. Uchino S, Kellum JA, Bellomo R, Doig GS, Morimatsu H, Morgera S, et al.; Beginning and Ending Supportive Therapy for the Kidney (BEST Kidney) Investigators: Acute renal failure in critically ill patients: a multinational, multicenter study. JAMA 2005; 294: 813-818.
4. ENCODE Project Consortium, Bernstein BE, Birney E, Dunham I, Green ED, Gunter C, Snyder M. An integrated encyclopedia of DNA elements in the human genome. Nature 2012; 489(7414):57-74.
5. Djebali S, Davis CA, Merkel A, Dobin A, Lassmann T, Mortazavi A, et al. Landscape of transcription in human cells. Nature 2012; 489(7414):101-8.
6. Lorenzen JM, Thum T. Circulating and urinary microRNAs in kidney disease. Clin J Am Soc Nephrol 2012; 7(9):1528-33.
7. Lorenzen JM, Haller H, Thum T. MicroRNAs as mediators and therapeutic targets in chronic kidney disease. Nat Rev Nephrol 2011; 7(5):286-94.
8. Lorenzen JM1, Martino F, Thum T. Epigenetic modifications in cardiovascular disease. Basic Res Cardiol 2012; 107(2):245.
9. Kumarswamy R1, Bauters C, Volkmann I, Maury F, Fetisch J, Holzmann A, et al. Circulating long noncoding RNA, LIPCAR, predicts survival in patients with heart failure. Circ Res 2014; 114(10):1569-75.
10. Martens-Uzunova ES, Böttcher R, Croce CM, Jenster G, Visakorpi T, Calin GA. Long noncoding RNA in prostate, bladder, and kidney cancer. Eur Urol 2014; 65(6):1140-51.
11. Faulhaber-Walter R, Hafer C, Jahr N et al. The Hannover Dialysis Outcome study: comparison of standard versus intensified extended dialysis for treatment of patients with acute kidney injury in the intensive care unit. Nephrol Dial Transplant 2009; 24(7):2179-86.
12. Fliser D, Kielstein JT: Technology Insight: treatment of renal failure in the intensive care unit with extended dialysis. Nat Clin Pract Nephrol 2006; 2: 32-39.
13. Vincent JL, Moreno R, Takala J et al: The SOFA (Sepsis-related Organ Failure Assessment) score to describe organ dysfunction/failure. On behalf of the Working Group on Sepsis-Related Problems of the European Society of Intensive Care Medicine. Intensive Care Med 1996; 22:707-710.
14. Knaus WA, Draper EA, Wagner DP et al: APACHE II: a severity of disease classification system. Crit Care Med 1985; 13:818-829.
15. Levy MM, Fink MP, Marshall JC et al: 2001 SCCM/ESICM/ACCP/ATS/SIS International Sepsis Definitions Conference. Intensive Care Med 2003; 29:530-538.
16. Bellomo R, Ronco C, Kellum JA et al: Acute renal failure - definition, outcome measures, animal models, fluid therapy and information technology needs: the Second International Consensus Conference of the Acute Dialysis Quality Initiative (ADQI) Group. Crit Care 2004; 8:R204-R212.
17. Ponting CP, Oliver PL, Reik W. Evolution and functions of long noncoding RNAs. Cell 2009; 136(4):629-41.
18. Zhao J, Sun BK, Erwin JA, Song JJ, Lee JT. Polycomb proteins targeted by a short repeat RNA to the mouse X chromosome. Science 2008; 322(5902):750-6.
19. Nagano T, Mitchell JA, Sanz LA, Pauler FM, Ferguson-Smith AC, Feil R, et al. The Air noncoding RNA epigenetically silences transcription by targeting G9a to chromatin. Science 2008; 322(5908):1717-20.
20. de Kok JB, Verhaegh GW, Roelofs RW, Hessels D, Kiemeney LA, Aalders TW, et al. DD3(PCA3), a very sensitive and specific marker to detect prostate tumors. Cancer Res 2002; 62(9):2695-8.
21. Hessels D, Klein Gunnewiek JM, van Oort I, Karthaus HF, van Leenders GJ, van Balken B, et al. DD3(PCA3)-based molecular urine analysis for the diagnosis of prostate cancer. Eur Urol 2003; 44(1):8-15; discussion 15-6.

## Claims

1. A method for predicting mortality of a test patient with acute kidney injury comprising
(a) detecting the expression level of the non-coding RNA (ncRNA) of SEQ ID NO: 1 in a blood sample obtained from said test patient with acute kidney injury,
(b) comparing said expression level of the ncRNA with the expression level of this ncRNA in a blood sample obtained from control patients with acute kidney injury or with a predetermined standard expression level of this ncRNA which was obtained on the basis of a blood sample from control patients with acute kidney injury, wherein
the control patients were alive at least about 4 weeks after diagnosis of the acute kidney injury, and a greater than 1.5-fold overexpression of the ncRNA in the test patient's blood sample as compared to the control patients' blood sample or as compared to the predetermined standard is indicative of an enhanced likelihood for future death of the test patient due to renal failure; and/or
(b') comparing said expression level of the ncRNA with the expression level of this ncRNA in a blood sample obtained from control patients with acute kidney injury or with a predetermined standard expression level of this ncRNA which was obtained on the basis of a blood sample from control patients with acute kidney injury, wherein
the control patients died from renal failure within about 4 weeks after diagnosis of acute kidney injury, and a greater than 1.5-fold underexpression of the ncRNA in the test patient's blood sample as compared to the control patients' blood sample or as compared to the predetermined standard is indicative of an enhanced likelihood for the long term survival of the test patient; and/or
(b") comparing said expression level of the ncRNA with the expression level of the ncRNA in a blood sample obtained from a healthy subject or with a predetermined standard expression level of this ncRNA which was obtained on the basis of at least one blood sample from a healthy subject,
wherein a greater than 5-fold overexpression of the ncRNA in the test patient's blood sample as compared to the healthy blood sample or as compared to the predetermined standard is indicative of an enhanced likelihood for future death of the test patient due to renal failure.

2. A method for determining whether a test patient has or is at risk of developing acute kidney injury comprising
(a) detecting the expression level of the ncRNA of SEQ ID NO: 1 in a blood sample obtained from said test patient, and
(b) comparing said expression level of the ncRNA with the expression level of the ncRNA in a blood sample obtained from a healthy subject or with a predetermined standard expression level of this ncRNA which was obtained on the basis of at least one blood sample from a healthy subject,
wherein a greater than 3-fold overexpression of the ncRNA in the test patient's blood sample as compared to the healthy blood sample or as compared to the predetermined standard is indicative for acute kidney injury.

3. The method of claim 1, wherein the patient(s) with acute kidney injury is/was (are/were) treated by renal replacement therapy when or after the blood sample was obtained.

4. The method of claim 3, wherein the renal replacement therapy is selected from hemodialysis, peritoneal dialysis, hemofiltration and renal transplantation.

5. The method of claim 1, 3 or 4, wherein the greater than 1.5-fold over- or underexpression as compared to the control patient's blood sample is at least greater than 2-fold over- or underexpression, preferably at least 2.5-fold over- or underexpression, and most preferably at least 3-fold over- or underexpression.

6. The method of claim 1, 3 or 4, wherein the greater than 5-fold over- or underexpression as compared to the blood sample of the healthy subject is at least greater than 10-fold over- or underexpression, preferably at least 20-fold over- or underexpression, and most preferably at least 30-fold over- or underexpression.

7. The method of claim 2, wherein the greater than 3-fold over- or underexpression as compared to the blood sample of the healthy subject is at least greater than 5-fold over- or underexpression, preferably at least 10-fold over- or underexpression, and most preferably at least 15-fold over- or underexpression.

8. The method of any one of the preceding claims, wherein the detection of the expression level of the ncRNA comprises
(i) quantitative PCR, preferably quantitative real time PCR, or
(ii) a template/RNA amplification method followed by determining the expression level of the ncRNA using a fluorescence- or luminescence-based quantification method.

9. The method of claim 8, wherein the primer sequences of SEQ ID NOs 2 and 3 are employed for the detection of the expression level of SEQ ID NO: 1.

10. The method of any one of the preceding claims, wherein the blood samples are plasma samples.

11. The method of any one of the preceding claims, wherein the method comprises prior to the detection of the expression level of the ncRNA a pre-amplification step of the RNA within the test patient's blood sample, the control patients' blood sample and/or the blood sample from a healthy subject.

12. The method of any one of the preceding claims, wherein the test patient and the control patient(s)/healthy subject are matched by one or more of age, sex, diabetes mellitus, renal failure etiology, and race.

13. The method of any one of the preceding claims, wherein the control patients/healthy subject are/is at least 3 control patients/healthy subjects, preferably a least 5 control patients/healthy subjects, and more preferably a least 10 control patients/healthy subjects.

14. A Kit for predicting mortality of a test patient with acute kidney injury and/or for determining whether a test patient has or is at risk of developing acute kidney injury, said kit comprising means for the detection of the expression level of the ncRNA of SEQ ID NO: 1 and instructions how to use the kit, wherein the means for the detection of the expression level of the ncRNA of SEQ ID NO: 1 comprise the primer pair of SEQ ID NOs 2 and 3.

## Patentansprüche

1. Verfahren zur Vorhersage der Sterblichkeit eines Testpatienten mit einer akuten Nierenschädigung, umfassend:
(a) Detektion des Expressionslevels der nicht-kodierenden RNA (ncRNA) der SEQ ID Nr. 1 in einer Blutprobe, die von dem Testpatienten mit einer akuten Nierenschädigung erhalten wurde,
(b) Vergleich des Expressionslevels der ncRNA mit dem Expressionslevel dieser ncRNA in einer Blutprobe, die von Kontrollpatienten mit einer akuten Nierenschädigung erhalten wurde oder mit einem vorbestimmten Standardexpressionslevel dieser ncRNA, das auf der Grundlage einer Blutprobe von Kontrollpatienten mit einer akuten Nierenschädigung erhalten wurde, wobei
die Kontrollpatienten mindestens etwa 4 Wochen nach der Diagnose der akuten Nierenschädigung am Leben waren und eine größer als 1,5-fache Überexpression der ncRNA in der Testpatienten-Blutprobe im Vergleich zur Kontrollpatienten-Blutprobe oder im Vergleich zum vorbestimmten Standard eine erhöhte Wahrscheinlichkeit eines künftigen Todes des Testpatienten aufgrund eines Nierenversagens anzeigt; und/oder
(b') Vergleich des Expressionslevels der ncRNA mit dem Expressionslevel dieser ncRNA in einer Blutprobe, die von Kontrollpatienten mit einer akuten Nierenschädigung erhalten wurde oder mit einem vorbestimmten Standardexpressionslevel dieser ncRNA, das auf der Grundlage einer Blutprobe von Kontrollpatienten mit einer akuten Nierenschädigung erhalten wurde, wobei
die Kontrollpatienten innerhalb von etwa 4 Wochen nach der Diagnose der akuten Nierenschädigung verstarben und eine größer als 1,5-fache Unterexpression der ncRNA in der Testpatienten-Blutprobe im Vergleich zur Kontrollpatienten-Blutprobe oder im Vergleich zum vorbestimmten Standard eine erhöhte Wahrscheinlichkeit eines langfristigen Überlebens des Testpatienten anzeigt; und/oder
(b") Vergleich des Expressionslevels der ncRNA mit dem Expressionslevel der ncRNA in einer Blutprobe, die von einem gesunden Individuum erhalten wurde oder mit einem vorbestimmten Standardexpressionslevel dieser ncRNA, das auf der Grundlage mindestens einer Blutprobe von einem gesunden Individuum erhalten wurde,
wobei eine größer als 5-fache Überexpression der ncRNA in der Testpatienten-Blutprobe im Vergleich zur gesunden Blutprobe oder im Vergleich zum vorbestimmten Standard eine erhöhte Wahrscheinlichkeit eines künftigen Todes des Testpatienten aufgrund von Nierenversagen anzeigt.

2. Verfahren zur Bestimmung, ob ein Testpatient eine akute Nierenschädigung hat oder ein Risiko dafür, eine solche zu entwickeln, umfassend:
(a) Nachweis des Expressionslevels der ncRNA der SEQ ID Nr. 1 in einer Blutprobe, die von dem Testpatienten erhalten wurde,
(b) Vergleich des Expressionslevels der ncRNA mit dem Expressionslevel der ncRNA in einer Blutprobe, die von einem gesunden Individuum erhalten wurde oder mit einem vorbestimmten Standardexpressionslevel dieser ncRNA, das auf der Grundlage mindestens einer Blutprobe von einem gesunden Individuum erhalten wurde,
wobei eine größer als 3-fache Überexpression der ncRNA in der Testpatienten-Blutprobe im Vergleich zur gesunden Blutprobe oder im Vergleich zum vorbestimmten Standard ein akutes Nierenversagens anzeigt.

3. Verfahren nach Anspruch 1, wobei der (die) Patient(en) mit einer akuten Nierenschädigung mittels einer Nierenersatztherapie behandelt wird/wurde (werden/wurden) zu dem Zeitpunkt als oder nachdem die Blutprobe erhalten wurde.

4. Verfahren nach Anspruch 3, wobei Nierenersatztherapie ausgewählt ist aus Hämodialyse, Peritonealdialyse, Hämofiltration und Nierentransplantation.

5. Verfahren nach einem der Ansprüche 1, 3 oder 4, wobei die größer als 1,5-fache Über- oder Unterexpression im Vergleich zur Kontrollpatienten-Blutprobe eine mindestens größer als 2-fache Über- oder Unterexpression ist, bevorzugt eine mindestens 2.5-fache Über- oder Unterexpression und besonders bevorzugt eine mindestens 3-fache Über- oder Unterexpression.

6. Verfahren nach einem der Ansprüche 1, 3 oder 4, wobei die größer als 5-fache Über- oder Unterexpression im Vergleich zur Blutprobe des gesunden Individuums eine mindestens größer als 10-fache Über- oder Unterexpression ist, bevorzugt eine mindestens 20-fache Über- oder Unterexpression und besonders bevorzugt eine mindestens 30-fache Über- oder Unterexpression.

7. Verfahren nach Anspruch 2, wobei die größer als 3-fache Über- oder Unterexpression im Vergleich zur Blutprobe des gesunden Individuums eine mindestens größer als 5-fache Über- oder Unterexpression ist, bevorzugt eine mindestens 10-fache Über- oder Unterexpression und besonders bevorzugt eine mindestens 15-fache Über- oder Unterexpression.

8. Verfahren nach einem der vorangegangen Ansprüche, wobei der Nachweis des Expressionslevels der ncRNA
(i) quantitative PCR, bevorzugt quantitative Echtzeit-PCR, oder
(ii) ein Template/RNA-Amplifikationsverfahren, gefolgt von der Bestimmung des Expressionslevels der ncRNA mittels eines Fluoreszenz- oder Lumineszenz-basierten Quantifizierungsverfahrens
umfasst.

9. Verfahren nach Anspruch 8, wobei die Primersequenzen der SEQ ID Nrn. 2 und 3 verwendet werden, um das Expressionslevel der SEQ ID Nr. 1 nachzuweisen.

10. Verfahren nach einem der vorangegangen Ansprüche, wobei die Blutproben Plasmaproben sind.

11. Verfahren nach einem der vorangegangen Ansprüche, wobei das Verfahren vor dem Nachweis des Expressionslevels der ncRNA einen Prä-Amplifikationsschritt der RNA in der Testpatienten-Blutprobe, der Kontrollpatienten-Blutprobe, und/oder der Blutprobe eines gesunden Individuums umfasst.

12. Verfahren nach einem der vorangegangen Ansprüche, wobei der Testpatient und der/die Kontrollpatient(en) das gesunde Individuum bezüglich einer oder mehrerer Eigenschaften ausgewählt aus Alter, Geschlecht, Diabetes mellitus, Nierenversagen-Ätiologie und Rasse aufeinander abgestimmt sind.

13. Verfahren nach einem der vorangegangen Ansprüche, wobei die Kontrollpatienten/das gesunde Individuum mindestens 3 Kontrollpatienten/gesunde Individuen sind, bevorzugt mindestens 5 Kontrollpatienten/gesunde Individuen und stärker bevorzugt mindestens 10 Kontrollpatienten/gesunde Individuen.

14. Kit zur Vorhersage der Sterblichkeit eines Testpatienten mit einer akuten Nierenschädigung und/oder zur Bestimmung, ob ein Testpatient eine akute Nierenschädigung hat oder ein Risiko dafür, eine solche zu entwickeln, wobei das Kit Mittel zum Nachweis des Expressionslevels der ncRNA der SEQ ID Nr. 1 umfasst und Instruktionen zur Verwendung des Kits, wobei die Mittel zur Detektion des Expressionslevels der ncRNA der SEQ ID Nr. 1 das Primerpaar der SEQ ID Nrn. 2 und 3 umfasst.

## Revendications

1. Méthode de prédiction de la mortalité d'un patient à tester souffrant d'une lésion rénale aiguë comprenant
(a) la détection du taux d'expression de l'ARN non codant (ARNnc) de SEQ ID NO : 1 dans un échantillon de sang obtenu à partir dudit patient à tester souffrant d'une lésion rénale aiguë,
(b) la comparaison dudit taux d'expression de l'ARNnc au taux d'expression de cet ARNnc dans un échantillon de sang obtenu à partir de patients témoins souffrant d'une lésion rénale aiguë ou à un taux d'expression standard prédéterminé de cet ARNnc qui a été obtenu sur la base d'un échantillon de sang provenant de patients témoins souffrant d'une lésion rénale aiguë, dans laquelle
les patients témoins étaient vivants au moins environ 4 semaines après le diagnostic de la lésion rénale aiguë et une surexpression de l'ARNnc supérieure à 1,5 fois dans l'échantillon de sang du patient à tester comparativement à l'échantillon de sang des patients témoins ou comparativement au standard prédéterminé indique une probabilité amplifiée d'un futur décès du patient à tester dû à l'insuffisance rénale ; et/ou
(b') la comparaison dudit taux d'expression de l'ARNnc au taux d'expression de cet ARNnc dans un échantillon de sang obtenu à partir de patients témoins souffrant d'une lésion rénale aiguë ou à un taux d'expression standard prédéterminé de cet ARNnc qui a été obtenu sur la base d'un échantillon de sang provenant de patients témoins souffrant d'une lésion rénale aiguë, dans laquelle
les patients témoins sont morts d'une insuffisance rénale en environ 4 semaines après le diagnostic d'une lésion rénale aiguë, et une sous-expression de l'ARNnc supérieure à 1,5 fois dans l'échantillon de sang du patient à tester comparativement à l'échantillon de sang des patients témoins ou comparativement au standard prédéterminé indique une probabilité amplifiée d'une survie à long terme du patient à tester ; et/ou
(b") la comparaison dudit taux d'expression de l'ARNnc au taux d'expression de l'ARNnc dans un échantillon de sang obtenu d'un sujet en bonne santé ou à un taux d'expression standard prédéterminé de cet ARNnc qui a été obtenu sur la base d'au moins un échantillon de sang provenant d'un sujet en bonne santé,
dans laquelle une surexpression de l'ARNnc supérieure à 5 fois dans l'échantillon de sang du patient à tester comparativement à l'échantillon de sang du sujet en bonne santé ou comparativement au standard prédéterminé indique une probabilité amplifiée d'un futur décès du patient à tester dû à l'insuffisance rénale.

2. Méthode de détermination si un patient à tester souffre de ou est à risque de développer une lésion rénale aiguë comprenant
(a) la détection du taux d'expression de l'ARNnc de SEQ ID NO : 1 dans un échantillon de sang obtenu dudit patient à tester, et
(b) la comparaison dudit taux d'expression de l'ARNnc au taux d'expression de l'ARNnc dans un échantillon de sang obtenu d'un sujet en bonne santé ou à un taux d'expression standard prédéterminé de cet ARNnc qui a été obtenu sur la base d'au moins un échantillon de sang provenant d'un sujet en bonne santé,
dans laquelle une surexpression de l'ARNnc supérieure à 3 fois dans l'échantillon de sang du patient à tester comparativement à l'échantillon de sang du sujet en bonne santé ou comparativement au standard prédéterminé indique une lésion rénale aiguë.

3. Méthode selon la revendication 1, dans laquelle le ou les patients souffrant d'une lésion rénale aiguë est/a (sont/ont) été traité(s) par une thérapie de remplacement rénal lorsque ou après que l'échantillon de sang a été obtenu.

4. Méthode selon la revendication 3, dans laquelle la thérapie de remplacement rénal est choisi parmi l'hémodialyse, la dialyse péritonéale, l'hémofiltration et la transplantation rénale.

5. Méthode selon la revendication 1, 3 ou 4, dans laquelle la surexpression ou la sous-expression supérieure à 1,5 fois comparativement à l'échantillon de sang du patient témoin est une surexpression ou une sous-expression au moins supérieure à 2 fois, de préférence une surexpression ou une sous-expression d'au moins 2,5 fois, et de façon préférée entre toutes une surexpression et une sous-expression d'au moins 3 fois.

6. Méthode selon la revendication 1, 3 ou 4, dans laquelle la surexpression ou la sous-expression supérieure à 5 fois comparativement à l'échantillon de sang du sujet en bonne santé est une surexpression ou une sous-expression au moins supérieure à 10 fois, de préférence une surexpression ou une sous-expression d'au moins 20 fois, et de façon préférée entre toutes une surexpression ou une sous-expression d'au moins 30 fois.

7. Méthode selon la revendication 2, dans laquelle la surexpression ou la sous-expression supérieure à 3 fois comparativement à l'échantillon de sang du sujet en bonne santé est une surexpression ou une sous-expression au moins supérieure à 5 fois, de préférence une surexpression et une sous-expression d'au moins 10 fois, et de façon préférée entre toutes une surexpression ou une sous-expression d'au moins 15 fois.

8. Méthode selon l'une quelconque des revendications précédentes, dans laquelle la détection du taux d'expression de l'ARNnc comprend
(i) une PCR quantitative, de préférence une PCR en temps réel quantitative, ou
(ii) une méthode d'amplification de matrice/ARN suivie de la détermination du taux d'expression de l'ARNnc en utilisant une méthode de quantification basée sur la fluorescence ou la luminescence.

9. Méthode selon la revendication 8, dans laquelle les séquences des amorces de SEQ ID NO : 2 et 3 sont employées pour la détection du taux d'expression de SEQ ID NO : 1.

10. Méthode selon l'une quelconque des revendications précédentes, dans laquelle les échantillons de sang sont des échantillons de plasma.

11. Méthode selon l'une quelconque des revendications précédentes, dans laquelle la méthode comprend avant la détection du taux d'expression de l'ARNnc une étape de pré-amplification de l'ARN dans l'échantillon de sang du patient à tester, l'échantillon de sang des patients témoins et/ou l'échantillon de sang provenant d'un sujet en bonne santé.

12. Méthode selon l'une quelconque des revendications précédentes, dans laquelle le patient à tester et le ou les patients témoins/le sujet en bonne santé correspondent par un ou plusieurs de l'âge, du genre, du diabète sucré, de l'étiologie de l'insuffisance rénale, et de la race.

13. Méthode selon l'une quelconque des revendications précédentes, dans laquelle les patients témoins/le sujet en bonne santé sont/est au moins 3 patients témoins/sujets en bonne santé, de préférence au moins 5 patients témoins/sujets en bonne santé, et de façon davantage préférée au moins 10 patient témoins/sujets en bonne santé.

14. Kit pour prédire la mortalité d'un patient à tester souffrant d'une lésion rénale aiguë et/ou pour déterminer si un patient à tester souffre de ou est à risque de développer une lésion rénale aiguë, ledit kit comprenant des moyens pour la détection du taux d'expression de l'ARNnc de SEQ ID NO : 1 et des instructions comment utiliser le kit, dans lequel les moyens pour la détection du taux d'expression de l'ARNnc de SEQ ID NO : 1 comprennent la paire d'amorces de SEQ ID NO : 2 et 3.
